(19) Europäisches Patentamt
European Patent Office
Office européen des brevets

(11) **EP 1 986 991 B1**

(12) **EUROPÄISCHE PATENTSCHRIFT**

(45) Veröffentlichungstag und Bekanntmachung des Hinweises auf die Patenterteilung:
**08.08.2012 Patentblatt 2012/32**

(51) Int Cl.:
*C07C 209/86* *(2006.01)*    *C07C 211/14* *(2006.01)*

(21) Anmeldenummer: **07712187.9**

(86) Internationale Anmeldenummer:
**PCT/EP2007/051227**

(22) Anmeldetag: **08.02.2007**

(87) Internationale Veröffentlichungsnummer:
**WO 2007/093555 (23.08.2007 Gazette 2007/34)**

(54) **VERFAHREN ZUR DESTILLATIVEN AUFTRENNUNG VON GEMISCHEN, ENTHALTEND MONOETHYLENGLYKOL UND DIETHYLENTRIAMIN**

PROCESS FOR THE DISTILLATIVE SEPARATION OF MIXTURES COMPRISING MONOETHYLENE GLYCOL AND DIETHYLENTRIAMINE

PROCEDE DE SEPARATION PAR DISTILLATION DE MELANGES CONTENANT DU MONOETHYLENE GLYCOL ET DE LA DIETHYLENE TRIAMINE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **14.02.2006 EP 06101641**

(43) Veröffentlichungstag der Anmeldung:
**05.11.2008 Patentblatt 2008/45**

(73) Patentinhaber: **BASF SE**
**67056 Ludwigshafen (DE)**

(72) Erfinder:
- **PICKENÄCKER, Karin**
  **68623 Lampertheim (DE)**
- **MELDER, Johann-Peter**
  **67459 Böhl-Iggelheim (DE)**
- **HOFFER, Bram Willem**
  **69120 Heidelberg (DE)**
- **KRUG, Thomas**
  **67550 Worms (DE)**
- **CAUWENBERGE, Gunther van**
  **B-9140 Temse (BE)**
- **PAPE, Frank-Friedrich**
  **67259 Kleinniedesheim (DE)**

(56) Entgegenhaltungen:
**WO-A-2005/037769    DD-A1- 217 509**
**US-A- 4 014 933**

- **LEE F-M: "EXTRACTIVE DISTILLATION: CLOSE-BOILING-POINT" CHEMICAL ENGINEERING, ACCESS INTELLIGENCE ASSOCIATION, ROCKVILLE, MA, US, Bd. 105, Nr. 12, November 1998 (1998-11), Seiten 112-116,118,12, XP000785548 ISSN: 0009-2460**

**Beschreibung**

Die Erfindung betrifft ein Verfahren zur destillativen Auftrennung von Gemischen, enthaltend Monoethylenglykol und Diethylentriamin.

Gemische, enthaltend Monoethylenglykol und Diethylentriamin fallen beispielsweise im Verfahren zur Herstellung von Ethylenaminen und Ethanolaminen durch hydrierende Aminierung von Monoethylenglykol (im Folgenden: MEG) in Gegenwart eines Katalysators an.

In bekannten Verfahren wird in der Regel ein Gemisch an Ethanolaminen und Ethylenaminen erhalten; hiervon sind insbesondere Ethylendiamin (im Folgenden: EDA) und Diethylentriamin (Bis(2-aminoethyl)amin; im Folgenden: DETA), wichtige Wertstoffe, unter anderem zur Verwendung als Lösungsmittel, Stabilisatoren, zur Synthese von Chelat-Bildnern, Kunstharzen, Arzneimitteln, Inhibitoren und grenzflächenaktiven Substanzen.

EDA wird insbesondere als Rohstoff für Fungizide und Insektizide eingesetzt.

DETA findet insbesondere Verwendung als Lösungsmittel für Farbstoffe und ist Ausgangsmaterial zur Herstellung von Ionenaustauschern, Schädlingsbekämpfungsmitteln, Antioxidantien, Korrosionsschutzmitteln, Komplexbildnern, Textilhilfsmitteln und Absorptionsmitteln für (saure) Gase.

Nicht-lineare Amine im Produktgemisch der Ethylenamine und Ethanolamine und insbesondere zyklische Amine, überwiegend Piperazin und Piperazinderivate, sind dagegen weniger begehrt bis unerwünscht.

Zur Herstellung von Ethylenaminen sind in der Literatur zahlreiche Verfahren beschrieben.

Gemäß PEP Report No. 138, "Alkyl Amines", SRI International, 03/1981, insbesondere Seiten 7, 8 13-16, 43-107, 113, 117, liefert die Umsetzung von Dichlorethan mit Ammoniak bei Molverhältnissen von 1:15 DETA mit einem Anteil an den gebildeten Ethylenaminen von größer 20 Gew.-%. Neben 40 Gew.-% EDA fallen jedoch 40 Gew.-% höhere Ethylenamine an.

Durch Aminierung von Monoethanolamin (im Folgenden: MEOA) mit Ammoniak (vgl. zum Beispiel den oben genannten PEP Report, US 4,014,933 (BASF AG)) kann die Bildung dieser höheren Ethylenamine (d. h. Ethylenaminen mit einem Siedepunkt über dem von Triethylentetramin (im Folgenden: TETA)) zugunsten von EDA weitgehend zurückgedrängt werden. Als Nebenprodukte fallen jedoch bei dieser Umsetzung Aminoethylethanolamin (im Folgenden: AEEA) und Piperazin (im Folgenden: PIP) an.

Ind. Eng. Chem. Prod. Res. Dev. 1981, 20, Seiten 399-407, (C.M. Barnes et al.) beschreibt die Ammonolyse von MEOA zu EDA an Nickel-Katalysatoren auf einem $SiO_2$-$Al_2O_3$-Mischträger. Zusatz von Wasser und der gepulverte Katalysator waren angeblich vorteilhaft bei der Erhöhung der Ausbeute an EDA.

Aus US 4,855,505 ist ein Verfahren zur Hydroaminierung von beispielsweise Monoethylenglykol mit beispielsweise Ammoniak in Gegenwart eines Katalysators bekannt, der 4 bis 40 Gew.-% Nickel oder Kobalt und 0,1 bis 5 Gew.-% Ruthenium, das als Lösung eines Rutheniumhalogenids eingeführt wurde, auf einem porösen Metalloxidträger, enthaltend mindestens 50 Gew.-% aktiviertes Aluminiumoxid, enthält. Der Katalysator wird beispielhaft in Form von Tabletten mit einer Länge und einem Durchmesser von etwa 3 mm eingesetzt.

Die in den beschriebenen Verfahren anfallenden Produktströme werden zur Reingewinnung der einzelnen Produkte, vor allem des besonders gewünschten EDAs und DETAs destillativ aufgetrennt. Problematisch ist hierbei, dass MEG und DETA ein Azeotrop bildet, das vom Druck nahezu unabhängig ist und daher durch Druckwechseldestillation nicht aufgetrennt werden kann. Die azeotrope Zusammensetzung liegt bei ca. 44 Gew.-% MEG und 56 Gew.-% DETA und hat bei 150 mbar einen Siedepunkt von 154°C, gegenüber dem Siedepunkt von reinem MEG von 144°C bzw. von reinem DETA von 142°C, jeweils bei dem oben aufgeführten Druck von 150 mbar.

Entsprechend war es Aufgabe der Erfindung, ein Verfahren zur Verfügung zu stellen, wonach MEG und DETA aus diese enthaltenden Gemischen destillativ abgetrennt werden können.

Die Lösung besteht in einem Verfahren zur destillativen Auftrennung eines Gemisches, enthaltend Monoethylenglykol und Diethylentriamin in einen Strom, der Monoethylenglykol enthält und weitgehend von Diethylenglykoltriamin frei ist, und einen Strom, der Diethylenglykoltriamin enthält und weitgehend von Monoethylenglykol frei ist, das dadurch gekennzeichnet ist, dass die Auftrennung durch Extraktivdestillation mit Triethylenglykol als selektivem Lösungsmittel für Diethylentetramin durchgeführt wird.

Es wurde überraschend gefunden, dass Triethylenglykol (im Folgenden: TEG) sich ausgezeichnet als selektives Lösungsmittel für die Auftrennung von MEG und DETA durch Extraktivdestillation eignet.

Erfindungsgemäß wird durch Einsatz von TEG als selektives Lösungsmittel für DETA ein Gemisch, enthaltend MEG und DETA, in einen Strom aufgetrennt, der MEG enthält, und dessen Anteil an DETA kleiner als 5 Gew.-%, bevorzugt kleiner als 0,1 Gew.-%, besonders bevorzugt kleiner als 10 ppm, ist und in einen Strom, der DETA enthält, und dessen Anteil an MEG kleiner ist als 2 Gew.-%, bevorzugt kleiner als 0,1 Gew.-%, besonders bevorzugt kleiner als 10 ppm.

In einer vorteilhaften Verfahrensvariante wird das MEG und DETA enthaltende Gemisch aus dem Reaktionsgemisch der hydrierenden Aminierung von MEG mit Ammoniak in Gegenwart eines heterogenen Katalysators erhalten, woraus gegenüber dem Azeotrop von MEG und DETA leichter und schwerer siedende Komponenten abgetrennt wurden.

Besonders vorteilhaft kann das MEG und DETA enthaltende Gemisch aus einem Verfahren zur Herstellung von Ethylenaminen und Ethanolaminen durch hydrierende Aminierung von MEG und Ammoniak in Gegenwart eines heterogenen Katalysators erhalten werden, wobei ein Katalysator eingesetzt wird, dessen Aktivmasse Ruthenium und Kobalt, daneben kein weiteres Metall der Gruppe VIII sowie kein Metall der Gruppe Ib enthält, und der als Katalysator-Formkörper vorliegt, der bei Kugelform oder Strangform jeweils einen Durchmesser von < 3 mm, bei Tablettenform eine Höhe von < 3 mm und bei allen anderen Geometrien jeweils einen Äquivalentdurchmesser L = 1/a' von < 0,70 mm aufweisen, wobei a' die externe Oberfläche per Volumeneinheit ($mm_s^2/mm_p^3$) ist, mit:

$$a' = \frac{Ap}{Vp}$$

wobei $A_p$ die externe Oberfläche des Katalysatorformkörpers ($mm_s^2$) und $V_p$ das Volumen des Katalysatorformkörpers ($mm_p^3$) ist.

Aus dem bei der hydrierenden Aminierung von MEG und Ammoniak in Gegenwart eines heterogenen Katalysators zur Herstellung von Ethylenaminen und Ethanolaminen anfallenden Produktgemisches wird insbesondere in einer ersten Trennsequenz zunächst überschüssiges Ammoniak, gebildetes Wasser und gegebenenfalls vorhandener Wasserstoff abgetrennt. Die hierfür benötigten Destillationskolonnen kann der Fachmann mit ihm geläufigen Methoden auslegen, insbesondere bezüglich der Zahl der Trennstufen, Rücklaufverhältnisse, usw. Hierbei anfallender Ammoniak und/oder anfallendes Wasser werden bevorzugt in die Umsetzung recycliert.

Das Reaktionsgemisch der hydrierenden Aminierung von MEG, aus dem bevorzugt in einer ersten Trennsequenz überschüssiges Ammoniak, gebildetes Wasser und gegebenenfalls vorhandener Wasserstoff abgetrennt wurden, wird anschließend in einer zweiten Trennsequenz in nicht umgesetztes MEG sowie MEOA, EDA, PIP, DETA, AEEA und höhere Ethylenamine aufgetrennt. Hierbei werden zunächst gegenüber dem Azeotrop von MEG und DETA niedriger sowie höher siedende Komponenten abgetrennt und anschließend das an MEG und DETA aufkonzentrierte Gemisch per Extraktivdestillation mit TEG als selektivem Lösungsmittel in einen MEG und einen DETA enthaltenden Strom aufgetrennt.

Insbesondere wird hierfür das Reaktionsgemisch der hydrierenden Aminierung von MEG, aus dem bevorzugt überschüssiges Ammoniak, gebildetes Wasser und gegebenenfalls vorhandener Wasserstoff abgetrennt wurden, in einer

- ersten Destillationseinheit KI in einen Kopfstrom, enthaltend die Komponenten des Reaktionsgemisches Ethylendiamin und Piperazin und einem Sumpfstrom, enthaltend die Komponenten des Reaktionsgemisches mit einem Siedepunkt größer als der Siedepunkt von Piperazin, aufgetrennt wird, wobei der Sumpfstrom

- einer zweiten Destillationskolonne KII zugeführt und darin in einen Kopfstrom, enthaltend Monoethylenglykol, Diethylentriamin und Monoethanolamin sowie einen Sumpfstrom, enthaltend gegenüber Monoethylenglykol und Diethylentriamin schwerer siedende Komponenten, aufgetrennt wird, wobei der Kopfstrom

- einer Extraktivdestillationskolonne KIII zugeführt wird, der auf gleicher Trennstufe oder Höhe ein Triethylenglykol als selektives Lösungsmittel für Diethylentriamin enthaltender Strom zugeführt wird, wobei in der Extraktivdestillationskolonne KIII über Sumpf ein mit Diethylentriamin beladener, das selektive Lösungsmittel Triethylenglykol enthaltender Strom und über Kopf ein Monoethylenglykol enthaltender, weitgehend von Diethylentriamin freier Strom gezogen wird.

Der Sumpfstrom aus der Extraktivdestillationskolonne KIII, enthaltend mit DETA beladenes selektives Lösungsmittel, wird bevorzugt einer Desorptionskolonne KIV zugeführt, und darin in einen DETA enthaltenden Kopfstrom und einen TEG enthaltenden Sumpfstrom aufgetrennt. Der TEG enthaltende Sumpfstrom aus der Extraktivdestillationskolonne KIV wird bevorzugt in die Extraktivdestillationskolonne KIII recycliert.

Die Zusammensetzung des in der Extraktivdestillation aufzutrennenden Stromes, d.h. des Zuführstromes zur Extraktivdestillationskolonne, beträgt bevorzugt 60 bis 90 Gew.-% MEG, 1,5 bis 6 Gew.-% DETA, 10 bis 30 Gew.-% MEOA und weniger als 1 Gew.-% Piperazin. Hierbei sind MEG und DETA bevorzugt in einem Gewichtsverhältnis im Bereich von 18:1 bis 42:1, enthalten.

Die Extraktivdestillation mit TEG als selektivem Lösungsmittel für DETA wird bevorzugt in der Weise betrieben, dass der Gewichtsanteil des Triethylenglykol enthaltenden Stromes oder der Triethylenglykol enthaltenden Ströme bezogen auf das Gewicht des Monoethylenglykol und Diethylentriamin enthaltenden Zuführstromes im Bereich von 1,5:1 bis 10:1, liegt.

Die Extraktivdestillationskolonne wird bevorzugt mit einer Anzahl von 5 bis 50 theoretischen Trennstufen, insbesondere 10 bis 30 theoretischen Trennstufen, besonders bevorzugt mit 20 theoretischen Trennstufen, ausgelegt, und bei einer Temperatur im Bereich von 60 bis 200°C, bevorzugt von 100 bis 180°C, und einem Druck von 0,01 bis 1 bar absolut, bevorzugt von 0,01 bis 0,5 bar absolut, betrieben.

Die Erfindung wird im Folgenden anhand einer Zeichnung und eines Ausführungsbeispiels näher erläutert.

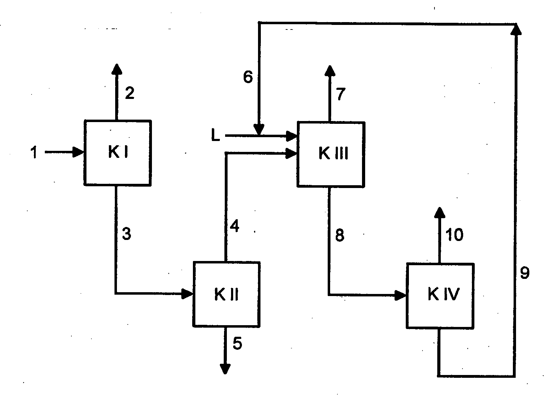

In der Zeichnung zeigt die einzige Figur 1 das Schema einer bevorzugten Anlage zur Durchführung des erfindungsgemäßen Verfahrens.

Ein Zuführstrom 1, enthaltend MEG und DETA, wird einer ersten Destillationseinheit KI zugeführt, und darin in einen Kopfstrom 2, enthaltend insbesondere EDA und PIP, und einen Sumpfstrom 3, enthaltend Komponenten mit einem Siedepunkt größer als der Siedepunkt von PIP, aufgetrennt. Der Sumpfstrom 3 der ersten Destillationseinheit KI wird einer zweiten Destillationseinheit KII zugeführt, und darin in einen Kopfstrom 4, enthaltend MEG und DETA, sowie einen Sumpfstrom 5 aufgetrennt, enthaltend gegenüber dem MEG und DETA schwerer siedende Komponenten, insbesondere AEEA, DEOA und Höhersieder.

Der Kopfstrom 4 aus der zweiten Destillationseinheit KII wird einer Extraktivdestillationskolonne KIII zugeführt, der auf gleicher Trennstufe oder höher ein TEG als selektives Lösungsmittel für DETA enthaltender Strom 6 zugeführt wird, und darin in einem mit DETA beladenes TEG enthaltenden Sumpfstrom, 8 und einen Kopfstrom 7 aufgetrennt, der überwiegend MEG und daneben MEOA enthält, und der von DETA weitgehend frei ist.

Der Sumpfstrom 8 aus der Extraktivdestillationskolonne KIII wird einer Desorptionskolonne KIV zugeführt, und darin in einen überwiegend DETA enthaltenden Kopfstrom 10 und einen Sumpfstrom 9 aufgetrennt, der TEG enthält, und der, in der in der Figur dargestellten bevorzugten Ausführungsvariante, in die Extraktivdestillationskolonne KIII recycliert wird.

Ausführungsbeispiel

Ein Reaktoraustrag aus der hydrierenden Aminierung von MEG mit Ammoniak in Gegenwart eines heterogenen Katalysators enthält nach Abtrennung von Ammoniak und Wasser 52 Gew.-% MEG, 21,5 Gew.-% MEOA, 17 Gew.-% EDA, 2 Gew.-% DETA und 2 Gew.-% AEEA, 3,5 Gew.-% Piperazin und 2 Gew.-% Höhersieder.

Dieses Gemisch wird als Zuführstrom 1 der ersten Destillationseinheit KI zugeführt und darin in einen Kopfstrom 2, enthaltend EDA und PIP und einen Sumpfstrom 3, enthaltend gegenüber PIP schwerer siedende Komponenten, aufgetrennt. Der Sumpfstrom 3 aus der ersten Destillationseinheit KI wird einer zweiten Destillationseinheit KII zugeführt und darin ein Schwersieder enthaltender Strom 5 abgetrennt sowie ein Kopfstrom 4, der als Zuführstrom in der Extraktivdestillationskolonne KIII zugeführt wird. Das Massenverhältnis von MEG und DETA im Zuführstrom 4 beträgt 28. Die Extraktivdestillationskolonne KIII wird bei einem Druck von 40 mbar und einem Rücklaufverhältnis von 1 betrieben. Sie ist mit 20 theoretischen Trennstufen ausgelegt und die Zuführung des MEG und DETA enthaltenden Stromes 4 erfolgt etwa mittig, bezogen auf die Trennstufen.

Das selektive Lösungsmittel für DETA, Strom 6, wird 1 bis 2 theoretische Trennstufen oberhalb des aufzutrennenden Gemisches 4 zugegeben. Der Massenstrom des selektives Lösungsmittel TEG enthaltenden Stromes 6 entspricht, bei einer Temperatur desselben von 25°C, der 3,8-fachen Menge des aufzutrennenden Stromes 4. Am Kopf der Extraktivdestillationskolonne KIII wird ein MEG und MEOA enthaltender Strom 7 abgetrennt, dessen DETA-Gehalt kleiner als 10 ppm ist. Am Sumpf der Extraktivdestillationskolonne KIII wird ein mit DETA beladener Strom des selektiven Lösungsmittels TEG abgezogen, der nahezu MEG-frei ist (MEG-Gehalt kleiner als 10 ppm). Strom 8 wird in der Desorptionskolonne KIV in einen DETA enthaltenden Kopfstrom 10 und einen Sumpfstrom 9 aufgetrennt, der das selektive Lösungsmittel TEG enthält, und der in die Extraktivdestillationskolonne KIII recycliert wird.

**Patentansprüche**

1. Verfahren zur destillativen Auftrennung eines Gemisches, enthaltend Monoethylenglykol und Diethylentriamin in einen Strom (7), der Monoethylenglykol enthält und in dem der Anteil an Diethylentriamin kleiner als 5 Gew.-%, bevorzugt kleiner als 0,1 Gew.-%, besonders bevorzugt kleiner als 10 ppm ist, und einen Strom (8), der Diethylentriamin enthält und in dem der Anteil an Monoethylenglykol kleiner als 2 Gew.-%, bevorzugt kleiner als 0,1 Gew.-%, besonders bevorzugt kleiner als 10 ppm ist, **dadurch gekennzeichnet, dass** die Auftrennung durch Extraktivdestillation mit Triethylenglykol als selektivem Lösungsmittel für Diethylentriamin durchgeführt wird.

2. Verfahren nach Anspruch 1, **dadurch gekennzeichnet, dass** das Monoethylenglykol und Diethylentriamin enthaltende Gemisch aus dem Reaktionsgemisch der hydrierenden Aminierung von Monoethylenglykol mit Ammoniak in Gegenwart eines heterogenen Katalysators erhalten wird, woraus gegenüber dem Azeotrop von Monoethylenglykol und Diethylentriamin leichter und schwerer siedende Komponenten abgetrennt wurden.

3. Verfahren nach Anspruch 2, **dadurch gekennzeichnet, dass** das Reaktionsgemisch der hydrierenden Aminierung von Monoethylenglykol (1), aus dem bevorzugt überschüssiges Ammoniak, gebildetes Wasser und gegebenenfalls vorhandener Wasserstoff abgetrennt wurden, in einer

    - ersten Destillationseinheit KI in einen Kopfstrom (2), enthaltend die Komponenten des Reaktionsgemisches Ethylendiamin und Piperazin und einem Sumpfstrom (3), enthaltend die Komponenten des Reaktionsgemisches mit einem Siedepunkt größer als der Siedepunkt von Piperazin, aufgetrennt wird, wobei der Sumpfstrom (3)

- einer zweiten Destillationskolonne KII zugeführt und darin in einen Kopfstrom (4), enthaltend Monoethylenglykol, Diethylentriamin und Monoethanolamin sowie einen Sumpfstrom (5), enthaltend gegenüber Monoethylenglykol und Diethylentriamin schwerer siedende Komponenten, aufgetrennt wird, wobei der Kopfstrom (4)

- einer Extraktivdestillationskolonne KIII zugeführt wird, der auf gleicher Trennstufe oder Höhe ein Triethylenglykol als selektives Lösungsmittel für Diethylentriamin enthaltender Strom (6) zugeführt wird, wobei in der Extraktivdestillationskolonne KIII über Sumpf ein mit Diethylentriamin beladener, das selektive Lösungsmittel Triethylenglykol enthaltender Strom 8 und über Kopf ein Monoethylenglykol enthaltender, weitgehend von Diethylentriamin freier Strom (7) gezogen wird.

4. Verfahren nach Anspruch 3, **dadurch gekennzeichnet, dass** der Sumpfstrom (8) aus der Extraktivdestillationskolonne KIII einer Desorptionskolonne KIV zugeführt wird, und darin in einen Diethylentriamin enthaltenden Kopfstrom (10) und einen Triethylenglykol enthaltenden Sumpfstrom (9) aufgetrennt wird.

5. Verfahren nach Anspruch 4, **dadurch gekennzeichnet, dass** der Sumpfstrom (9) aus der Desorptionskolonne KIV in die Extraktivdestillationskolonne KIII recycliert wird.

6. Verfahren nach einem der Ansprüche 3 bis 5, **dadurch gekennzeichnet, dass** der Zuführstrom (4) zur Extraktivdestillationskolonne KIII Monoethylenglykol und Diethylentriamin in einem Gewichtsverhältnis im Bereich von 18:1 bis 42:1 enthält.

7. Verfahren nach Anspruch 6, **dadurch gekennzeichnet, dass** der Zuführstrom (4) zur Extraktivdestillationskolonne KIII 60 bis 90 Gew.-% Monoethylenglykol, 1,5 bis 6 Gew.-% Diethylentriamin, 10 bis 30 Gew.-% Monoethanolamin und weniger als 1 Gew.-% Piperazin, enthält.

8. Verfahren nach einem der Ansprüche 3 bis 7, **dadurch gekennzeichnet, dass** der Gewichtsanteil des Triethylenglykol enthaltenden Stromes (6) oder der Triethylenglykol enthaltenden Ströme (6) und (9), bezogen auf das Gewicht des Monoethylenglykol und Diethylentriamin enthaltenden Zuführstromes (4) im Bereich von 1,5:1 bis 10:1, liegt.

9. Verfahren nach einem der Ansprüche 3 bis 8, **dadurch gekennzeichnet, dass** die Extraktivdestillationskolonne KIII bei einer Temperatur im Bereich von 60 bis 200°C, bevorzugt von 100 bis 180°C, und einem Druck von 0,01 bis 1 bar absolut, bevorzugt von 0,01 bis 0,5 bar absolut, betrieben wird, und eine Anzahl von 5 bis 50 theoretischen Trennstufen, bevorzugt von 10 bis 30 theoretischen Trennstufen, besonders bevorzugt 20 theoretische Trennstufen, aufweist.

**Claims**

1. A process for distillatively separating a mixture comprising monoethylene glycol and diethylenetriamine into a stream (7) which comprises monoethylene glycol and in which the proportion of diethylenetriamine is less than 5% by weight, preferably less than 0.1% by weight, more preferably less than 10 ppm, and a stream (8) which comprises diethylenetriamine and in which the proportion of monoethylene glycol is less than 2% by weight, preferably less than 0.1% by weight, more preferably less than 10 ppm, which comprises performing the separation by extractive distillation with triethylene glycol as a selective solvent for diethylenetriamine.

2. The process according to claim 1, wherein the mixture comprising monoethylene glycol and diethylenetriamine is obtained from the reaction mixture of the hydrogenating amination of monoethylene glycol with ammonia in the presence of a heterogeneous catalyst, from which lower- and higher-boiling components relative to the azeotrope of monoethylene glycol and diethylenetriamine have been removed.

3. The process according to claim 2, wherein the reaction mixture of the hydrogenating amination of monoethylene glycol (1), from which excess ammonia, water formed and any hydrogen present have preferably been removed, is separated in a

- first distillation unit KI into a top stream (2) comprising the ethylenediamine and piperazine components of the reaction mixture and a bottom stream (3) comprising the components of the reaction mixture having a boiling point greater than the boiling point of piperazine, the bottom stream (3) being fed to

- a second distillation column KII and separated therein into a top stream (4) comprising monoethylene glycol, diethylenetriamine and monoethanolamine, and a bottom stream (5) comprising components having a higher boiling point than monoethylene glycol and diethylenetriamine, the top stream (4) being fed to

- an extractive distillation column KIII to which is fed, at the same separating stage or height, a stream (6) comprising triethylene glycol as a selective solvent for diethylenetriamine, a diethylenetriamine-laden stream (8) comprising the selective solvent triethylene glycol being removed via the bottom, and a monoethylene glycol-comprising stream (7) freed substantially of diethylenetriamine being removed via the top in the extractive distillation column KIII.

4. The process according to claim 3, wherein the bottom stream (8) from the extractive distillation column KIII is fed to a desorption column KIV and separated therein into a top stream (10) comprising diethylenetriamine and a bottom stream (9) comprising triethylene glycol.

5. The process according to claim 4, wherein the bottom stream (9) from the desorption column KIV is recycled into the extractive distillation column KIII.

6. The process according to any of claims 3 to 5, wherein the feed stream (4) to the extractive distillation column KIII comprises monoethylene glycol and diethylenetriamine in a weight ratio in the range from 18:1 to 42:1.

7. The process according to claim 6, wherein the feed stream (4) to the extractive distillation column KIII comprises from 60 to 90% by weight of monoethylene glycol, from 1.5 to 6% by weight of diethylenetriamine, from 10 to 30% by weight of monoethanolamine and less than 1% by weight of piperazine.

8. The process according to any of claims 3 to 7, wherein the proportion by weight of the stream (6) comprising triethylene glycol or of the streams (6) and (9) comprising triethylene glycol, based on the weight of the feed stream (4) comprising monoethylene glycol and diethylenetriamine, is in the range from 1.5:1 to 10:1.

9. The process according to any of claims 3 to 8, wherein the extractive distillation column KIII is operated at a temperature in the range from 60 to 200°C, preferably from 100 to 180°C, and a pressure of from 0.01 to 1 bar absolute, preferably from 0.01 to 0.5 bar absolute, and has a number of from 5 to 50 theoretical plates, preferably from 10 to 30 theoretical plates, more preferably 20 theoretical plates.

**Revendications**

1. Procédé pour le fractionnement par distillation d'un mélange, contenant du monoéthylèneglycol et de la diéthylènetriamine, en un courant (7) qui contient du monoéthylèneglycol et dans lequel la proportion de diéthylènetriamine est inférieure à 5 % en poids, de préférence inférieure à 0,1 % en poids, de façon particulièrement préférée, inférieure à 10 ppm, et un courant (8) qui contient de la diéthylènetriamine et dans lequel la proportion de monoéthylèneglycol est inférieure à 2 % en poids, de préférence inférieure à 0,1 % en poids, de façon particulièrement préférée, inférieure à 10 ppm, **caractérisé en ce que** le fractionnement est effectué par distillation extractive avec du triéthylèneglycol en tant que solvant sélectif pour la diéthylènetriamine.

2. Procédé selon la revendication 1, **caractérisé en ce que** le mélange contenant du monoéthylèneglycol et de la diéthylènetriamine est obtenu à partir du mélange réactionnel de l'amination hydrogénante de monoéthylèneglycol avec de l'ammoniac en présence d'un catalyseur hétérogène, dont ont été séparés les composants à plus bas et à plus haut point d'ébullition par rapport à l'azéotrope de monoéthylèneglycol et diéthylènetriamine.

3. Procédé selon la revendication 2, **caractérisé en ce que** le mélange réactionnel de l'amination hydrogénante de monoéthylèneglycol (I), duquel ont été séparé de préférence l'ammoniac en excès, l'eau formée et l'hydrogène éventuellement présent, est fractionné

   - dans une première unité de distillation KI en un courant de tête (2), contenant les composants du mélange réactionnel éthylènediamine et pipérazine et un courant de fond (3), contenant les composants du mélange réactionnel ayant un point d'ébullition supérieur au point d'ébullition de la pipérazine, le courant de fond (3)
   - étant envoyé dans une deuxième colonne de distillation KII et étant fractionné dans celle-ci en un courant de tête (4), contenant du monoéthylèneglycol, de la diéthylènetriamine et de la monoéthanolamine, ainsi qu'en un courant de fond (5), contenant des composants à plus haut point d'ébullition par rapport au monoéthylèneglycol et à la diéthylènetriamine, le courant de tête (4)
   - étant envoyé à une colonne de distillation extractive KIII, à laquelle est envoyé au niveau du même étage de séparation ou à la même hauteur un courant (6) contenant du triéthylèneglycol en tant que solvant sélectif pour la diéthylènetriamine, dans la colonne de distillation extractive KIII un courant (8) chargé de diéthylènetriamine, contenant le solvant sélectif triéthylèneglycol, étant soutiré par le fond et un courant (7) contenant du monoéthylèneglycol, essentiellement exempt de diéthylènetriamine, étant déchargé par la tête.

4. Procédé selon la revendication 3, **caractérisé en ce que** le courant de fond (8) provenant de la colonne de distillation extractive KIII est envoyé à une colonne de désorption KIV, et dans cette colonne est fractionné en un courant de tête (10) contenant de la diéthylènetriamine et un courant de fond (9) contenant du triéthylèneglycol.

5. Procédé selon la revendication 4, **caractérisé en ce que** le courant de fond (9) provenant de la colonne de désorption KIV est recyclé dans la colonne de distillation extractive KIII.

6. Procédé selon l'une quelconque des revendications 3 à 5, **caractérisé en ce que** le courant d'alimentation (4) conduisant à la colonne de distillation extractive KIII contient du monoéthylèneglycol et de la diéthylènetriamine en un rapport pondéral dans la plage de 18:1 à 42:1.

7. Procédé selon la revendication 6, **caractérisé en ce que** le courant d'alimentation (4) conduisant à la colonne de distillation extractive KIII contient 60 à 90 % en poids de monoéthylèneglycol, 1,5 à 6 % en poids de diéthylènetriamine, 10 à 30 % en poids de monoéthanolamine et moins de 1 % en poids de pipérazine.

8. Procédé selon l'une quelconque des revendications 3 à 7, **caractérisé en ce que** la proportion pondérale du courant (6) contenant du triéthylèneglycol ou des courants (6) et (9) contenant du triéthylèneglycol se situe dans la plage de 1,5:1 à 10:1, par rapport au poids du courant d'alimentation (4) contenant du monoéthylèneglycol et de la diéthylènetriamine.

9. Procédé selon l'une quelconque des revendications 3 à 8, **caractérisé en ce que** la colonne de distillation extractive KIII est utilisée à une température dans la plage de 60 à 200 °C, de préférence de 100 à 180 °C, et sous une pression absolue de 0,01 à 1 bar, de préférence sous une pression absolue de 0,01 à 0,5 bar, et comporte un nombre de 5 à 50 étages de séparation théoriques, de préférence de 10 à 30 étages de séparation théoriques, de façon particulièrement préférée 20 étages de séparation théoriques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4014933 A

- US 4855505 A

**In der Beschreibung aufgeführte Nicht-Patentliteratur**

- Alkyl Amines. *PEP Report No. 138,* 7, 8 13-1643-107113, 117

- **C.M. BARNES.** *Ind. Eng. Chem. Prod. Res. Dev.,* 1981, vol. 20, 399-407